# EUROPÄISCHE PATENTSCHRIFT

(11) **EP 0 470 961 B1**
(45) Veröffentlichungstag und Bekanntmachung des Hinweises auf die Patenterteilung: **15.12.1993**
(21) Anmeldenummer: 90903820.0
(22) Anmeldetag: 03.03.1990
(51) Int. Cl.: G01N 1/28, G01N 33/20

(54) **PROBEN-AUFBEREITUNGSSYSTEM FÜR EISEN- UND STAHLPROBEN**
SAMPLE-PREPARATION SYSTEM FOR IRON AND STEEL SAMPLES
SYSTEME DE PREPARATION D'ECHANTILLONS DE FER ET D'ACIER

(43) Veröffentlichungstag der Anmeldung: 19.02.1992
(73) Patentinhaber: Herzog Maschinenfabrik GmbH & Co., D-49086 Osnabrück (DE)
(72) Erfinder: HERZOG, Helga, D-4512 Wallenhorst (DE); HAWICKHORST, Günther, D-4550 Bramsche (DE)
(74) Vertreter: Hanewinkel, Lorenz, Dipl.-Phys.
(86) Internationale Anmeldenummer: EP9000359
(87) Internationale Veröffentlichungsnummer: WO9114166

(56) Entgegenhaltungen:
- DE-A- 3 722 180
- DE-C- 3 344 944
- FR-A- 2 406 822
- Patent Abstracts of Japan, volume 8, No. 163, P290 Abstract of 59-60241

## Beschreibung

Die Erfindung bezieht sich auf ein System, mit dem Eisen- und Stahlproben für die Probenanalyse aufbereitet werden.

Zur Prozeßkontrolle und Qualitätssicherung werden in der Stahlindustrie während des Prozeßablaufes und von den Fertigprodukten regelmäßig Proben entnommen. Die chemische Zusammensetzung dieser Proben wird mittels optischer Emissions-, Röntenfluoreszens- und Verbrennungsanalyse bestimmt.

Die Proben sollen entsprechend den Erfordernissen der Analysengeräte Vollautomatisch und reproduzierbar vorbereitet werden können, was z. B. das Erzeugen einer planen Oberfläche oder das Ausstanzen von kleinen Stücken sein kann.

Aus der Druckschrift DE-C-3 344 944 ist ein Aufbereitungssystem gemäß dem ersten Teil des Anspruchs 1 bekannt, bei dem über lose aneinandergereihte Statronen und Einrichtungen die jeweiligen Verfahrensschritte durchgeführt werden.

Aufgabe der Erfindung ist es, ein Probenaufbereitungssystem für Eisen- und Stahlproben zu schaffen, bei dem die für eine vollautomatische und reproduzierbare Probenvorbereitung einzusetzenden Bearbeitungsmaschinen in variabler Weise zu einer vollautomatischen Einheit zusammengefaßt sind und das Erstellen qualitätsmäßig einwandfreier Proben ermöglichen.

Diese Aufgabe wird erfindungsgemäß durch ein Aufbereitungssystem mit den Merkmalen des Patentanspruches 1 gelöst, wobei noch die in den Unteransprüchen 2 bis 5 aufgeführten Gestaltungsmerkmale vorteilhafte Weiterbildungen der Aufgabenlösung darstellen.

Eine weitere Aufgabe der Erfindung wird in der Schaffung einer einfach aufgebauten und sicher wirkenden Spannvorrichtung für die Proben gesehen, die in kompakter Bauweise bei kurzen Bewegungswegen und geringem Kraftaufwand einen großen Spannhub und eine hohe Spannkraft ermöglicht.
Diese Aufgabe wird durch das Aufbereitungssystem mit den Merkmalen des Patentanspruches 6 gelöst; die sich daran anschließenden Unteransprüche 7 bis 16 ergeben vorteilhafte und förderliche Gestaltungsmerkmale in der Ausgestaltung der Spannvorrichtung.

Das erfindungsgemäße Proben-Aufbereitungssystem setzt sich aus einer modularen Baueinheit zusammen, mit einer Schleifmaschine, einer Stanzeinrichtung und einer Trennmaschine bei dem die drei Module für die Proben-Ein- und -Ausgabe und die Proben-Übergabe durch Transportsysteme miteinander verknüpft sind, was ein automatisches Bewegen der Proben in das Aufbereitungssystem und innerhalb desselben ergibt.

Die einzelnen Module sind mit Bearbeitungsstationen ausgerüstet, die eine qualitätsmäßig hochwertige Vorbereitung der Proben durchführen.

Weiterhin ist die Schleifmaschine mit einer kompakt aufgebauten Spannvorrichtung für die zu schleifenden Proben ausgestattet, die bei kurzen Bewegungswegen und geringem Krafteinsatz einen verhältnismäßig großen Spannhub und eine hohe Spannkraft ihrer Spannbacken besitzt.

Die Spannvorrichtung ist einfach aufgebaut und arbeitet in zwei Spannstufen, wobei die erste Spannstufe durch axiales Verdrehen eines Spanntellers ein Bewegen der Spannbacken gegeneinander ergibt und die zweite Spannstufe durch Druckbeaufschlagung des Spanntellers erreicht wird; somit werden die Spannbacken zunächst auf einem größeren Hub vorgespannt und anschließend auf einem wesentlich kleineren Hub nachgespannt.

Auf den Zeichnungen ist ein Ausführungsbeispiel der Erfindung dargestellt, welches nachfolgend näher erläutert wird. Es zeigt:
- Fig. 1: eine schematische Draufsicht auf ein modulares, aus einer Schleifmaschine, einer Stanzeinrichtung und einer Trennmaschine bestehendes Probenaufbereitungssystem,
- Fig. 2: eine schematische Draufsicht auf das Probenaufbereitungssystem mit Proben-Ein- und -Ausgaben sowie Proben-Übergaben und einer Spannvorrichtung,
- Fig. 3: eine schematische Draufsicht auf das Probenaufbereitungssystem mit Bearbeitungsstationen der unteren Ebene der Stanzeinrichtung,
- Fig. 4: eine schematische Draufsicht des Probenaufbereitungssystems mit in der oberen Ebene angeordneten Bearbeitungsstationen,
- Fig. 5: eine schematische Vorderansicht der Schleifmaschine mit verfahrbarer Spannvorrichtung und Grob- und Feinschleifeinrichtung,
- Fig. 6 bis 8: verschiedene Probenarten,
- Fig. 9: einen senkrechten Schnitt durch die Spannvorrichtung gemäß Schnittlinie III-III in Fig. 12, in der geöffneten Stellung der Spannbacken,
- Fig. 10: einen senkrechten Schnitt durch die Spannvorrichtung in der ersten Spannstufe der Spannbacken,
- Fig. 11: einen senkrechten Schnitt durch die Spannvorrichtung in der zweiten Spannstufe der Spannbacken,
- Fig. 12: einen waagerechten Schnitt durch die Spannvorrichtung gemäß Schnittlinie I-I in Fig. 9,
- Fig. 13: einen waagerechten Schnitt durch die Spannvorrichtung entsprechend der Schnittlinie II-II in Fig. 9,
- Fig. 14: eine Seitenansicht im teilweisen Schnitt der Spannvorrichtung.

Das Probenaufbereitungssystem besteht aus den Modulen Schleifmaschine (1), Stanzeinrichtung (2) und Trennmaschine (3).

Die Proben (4, 5, 6) werden dem System über Transporteinrichtungen (7, 8, 9, 10, 11, 12) zugeführt sowie innerhalb des Systems von einem Modul zum nächsten übergeben.

Weiterhin kann die Proben-Übergabe innerhalb des Aufbereitungssystems durch einen integrierten Roboter (12) vorgenommen werden. Für die Ein- und Ausgabe sowie Übergabe der Proben (4 bis 6) lassen sich als Transporteinrichtungen (7 bis 11) Transportbänder vorsehen.

Das zentrale Modul bildet die Schleifmaschine (1), an die, je nach Probenart, die Module Stanzeinrichtung (2) für Laschenproben (4) (auch Lolly Pop-Proben genannt) und Trennmaschine (3) für zylindrische und konische Proben (5, 6) angekoppelt werden können.

Die zentrale Entstaubung des Systems erfolgt über einen Entstaubungsanschluß (13) der Schleifmaschine (1), wodurch das System in unmittelbarer Nähe der empfindlichen Analysengeräte aufgestellt werden kann.

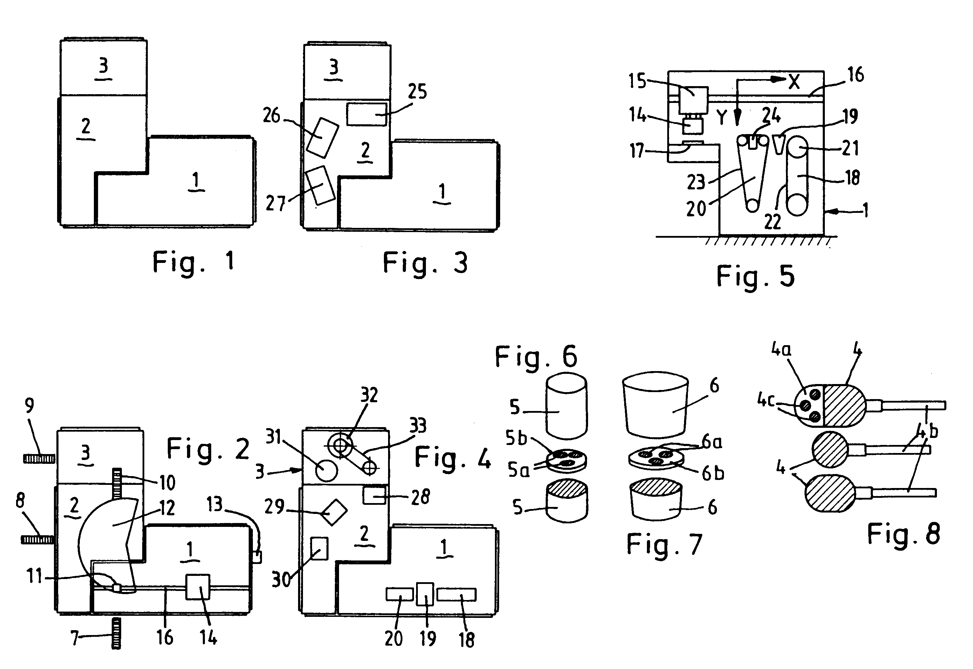

Die Schleifmaschine (1) besitzt eine Spannvorrichtung (14), die mittels eines Supports (15) an Führungen (16) durch einen Servomotorantrieb in X-Richtung bewegt wird (vgl. Fig. 5).

Die Spannvorrichtung (14) wird über einen zweiten Servomotorantrieb in Y-Richtung bewegt und hat für diese Bewegung innerhalb des Supports (15) die Führung.

Die Probe (4, 5, 6) wird von einer Probenauflage (17) durch die Spannvorrichtung (14) aufgenommen, gespannt und auf einer Grobschleifeinrichtung (18) vorgeschliffen.

Danach erfolgt die Kühlung der Probe (4, 5, 6) in der Kühleinrichtung (19) mittels eines flüssigen Mediums, z. B. Wasser, und dann der Feinschleifvorgang auf einer Feinschleifeinrichtung (20). Während dieser Zeit wird die Probe (4, 5, 6) zusätzlich durch ein gasförmiges Medium, z. B. Druckluft, gekühlt. Dieser Ablauf wird durch ein Programm gesteuert. Die Reihenfolge des Ablaufes kann in Voreinstellungen beliebig geändert und damit den Erfordernissen der jeweiligen Probenart angepaßt werden.

Bei rißempfindlichen Proben wird ein Programm gewählt, das die Probe mit Druckluft vorkühlt und dann mit Wasser auf Raumtemperatur nachkühlt.

Das Schleifen auf der Grobschleifeinrichtung (18) erfolgt in der Weise, daß die Probe (4, 5, 6) in der Spanneinrichtung (14) fest gespannt oszillierend über ein Kontaktrad (21) bewegt wird, welches vom Schleifband (22) umschlungen ist. Dieses Kontaktrad (21) treibt gleichzeitig das Schleifband (22) an. Während der oszillatorischen Bewegung erfolgt die schrittweise Zustellung in Y-Richtung, bis die voreingestellte Schleiftiefe erreicht ist. Dieser Schleifvorgang erfolgt sehr schnell (ca. 3 Sekunden für eine übliche Abschleiftiefe von 0,6 mm), wobei die Probe (4, 5, 6) nicht mit ihrer ganzen Fläche, sondern nur mit einem schmalen Radiusbereich des Kontaktrades (21) und somit des Schleifbandes (22) in Berührung kommt. Dieser Umstand verhindert eine zu starke Erwärmung der Probe (4, 5, 6). Eine starke Erwärmung kann zur Folge haben, daß Risse in der Probenoberfläche entstehen oder vorhandene Mikrorisse aufklaffen, in die während der Flüssigkeitskühlung Wasser eindringen kann, was sich nachteilig bei der anschließenden Analyse auswirkt.

An Stelle des Schleifbandes (22) kann auch eine Topfschleifscheibe für die Grobschleifeinrichtung (18) eingesetzt werden. Diese Topfschleifscheibe ist an einer vertikal stehenden Schleifspindel befestigt.

Aufgrund der hohen Standzeit des Schleiftopfes ist dieses Verfahren insbesondere für das Schleifen von Eisenproben geeignet.

Das Schleifen auf der Feinschleifeinrichtung (20) erfolgt in der Weise, daß die Probe (4, 5, 6), die in der Spannvorrichtung (14) fest gespannt ist, über das Feinschleifband (23) gebracht wird und dabei die Zustellung in Y-Richtung erfolgt.

Ein unter dem Schleifband (23) federnd gelagerter Schleiftisch (24) erzeugt dabei die für das Schleifen erforderliche Gegenkraft.

Dieses Schleifverfahren erzeugt die für die Analyse erforderliche, reproduzierbare Oberfläche.

Der kurze Kontakt mit dem Schleifband (23) (ca. 2 Sekunden) erbringt keine wesentliche Erwärmung der Probe (4, 5, 6) und verringert die Möglichkeit der Materialübertragung von Restprobenmaterial auf die Analysenfläche der Probe (4, 5, 6) wesentlich.

Dieser Umstand ist für eine unverfälschte Analyse von entscheidender Bedeutung.

Die Stanzeinrichtung (2) besitzt einen zentralen Roboter (12), der die Proben (4, 5, 6) in die einzelnen Bearbeitungsstationen (25, 26, 27, 28, 29, 30) übergibt. Diese Bearbeitungsstationen (25 bis 30) sind halbkreisförmig um den Roboter (12) angeordnet. Der Roboter (12) kann die Proben (4, 5, 6) von den Transporteinrichtungen (8, 9, 10) aufnehmen und sie auch wieder über den gleichen Weg aus dem System ausschleusen.

Die Übergabe der Proben (4, 5, 6) in die Schleifmaschine (1) erfolgt durch die Transporteinrichtung (11), die die Proben (4, 5, 6) von Modul 2 nach Modul 1 bringt (Fig. 2).

In der Stanzeinrichtung (2) sind als Bearbeitungsstationen in der unteren Ebene eine Sandstrahleinheit (26), eine induktive Erwärmstation (27) und ein Probenbrecher (25) und in der oberen Ebene drei Stanzen (28 bis 30) angeordnet.

Die Sandstrahleinheit (26) besitzt zwei schräg angestellte Düsen, welche das Strahlmedium in einen Preßluftstrom zur Probenlasche (4a) fördern und diese von der Zunderschicht befreien. Dadurch werden Verfälschungen bei der Verbrennungsanalyse weitgehendst verhindert.

Mit der induktiven Erwärmstation (27) wird die Probenlasche (4a) in einer Spule induktiv auf 700 bis 900 Grad C erwärmt. Dadurch lassen sich auch die Stahlqualitäten mit hohen Kohlenstoffgehalten und hochlegierte Stähle stanzen.

Das Einbringen der Probenlasche (4a) in die Spule übernimmt der Roboter (12). Ein Überhitzen der Probe (4a) wird durch eine Temperaturüberwachung verhindert.

Eisenproben sind nicht stanzbar. Um Probenstücke für die Verbrennungsanalyse zu erhalten werden entweder die Probe selbst oder ein Teil derselben gebrochen, hierfür ist der Probenbrecher (25) vorgesehen. Die abzubrechenden Probenstücke können z. B. eine angegossene Lasche (4a) oder ein Probenstiel (4b) sein. In einer der Stanzen (28, 29 oder 30) kann ein Werkzeug eingesetzt werden, das die Lasche (4a) oder den Stiel (4b) von der Probe (4) abbricht. Das Brechen in kleine Stücke übernimmt dann der eingebaute Probenbrecher (25), in welchen die Lasche (4a) oder der Stiel (4b) durch den Roboter (12) eingegeben wird. Der Brechvorgang wird zwischen zwei Hartmetallbacken vorgenommen, die sich in kreisförmigen Bewegungen gegeneinander bewegen. Der Antrieb erfolgt hydraulisch. Die Spaltweite der Brechbacken ist einstellbar, damit die Korngröße der gebrochenen Teile variiert werden kann.

In der ersten Stanze (28) wird die Lasche (4a) von der Probe (4) abgeschert. Dieses ist bei Laschenproben (4) erforderlich, wenn die Proben (4) in die Kassetten der Röntgenfluoreszensspektrometer eingesetzt werden sollen.

Für die Verbrennungsanalyse von Kohlenstoff, Stickstoff und Schwefel werden Probenstücke (4c, 6a, 5a) von 0,5 bis 1 g benötigt. Hierfür werden kleine Ronden (4c, 6a, 5a) aus der Probenlasche (4a) bzw. aus Probenscheiben (5b, 6b) ausgestanzt. Die Ronden (4c, 5a, 6a) werden mittels eines Injektors in einer Rohrleitung mit einem gasförmigen Medium zu einer Auffangeinrichtung, z. B. einem Magazin, oder direkt zu dem Analysengerät gefördert. Die Ronden (4c, 5a, 6a) werden in der zweiten Stanze (29) ausgestanzt.

Die dritte Stanze (30) schert den Stiel (4b) ab, da der Stiel (4b) an der Probe (4) die weitere Handhabung erschweren würde. Der abgescherte Stiel (4b) wird in einem Abfallbehälter aufgefangen.

Die Trennmaschine (3) dient dazu,aus zylindrischen und konischen Probenkörpern (5, 6) für die weitere Bearbeitung in der Stanzeinrichtung (2) und der Schleifmaschine (1) Probenscheiben (5b, 6b) herauszuschneiden. Die Proben (5, 6) werden automatisch in einem rotierenden Spannfutter (31) gespannt und dann mit einer Trennschleifscheibe (32) der Trennmaschine (3) durchgetrennt. Durch das Rotieren der Probe (5, 6) während des Trennvorganges wird eine plane Oberfläche erzeugt. Außerdem verringert die Rotation die Erwärmung der Probe (5, 6), da die Trennschleifscheibe (32) nicht über die Mitte der Probe (5, 6) hinaus im Eingriff ist und die Eingriffposition ständig wechselt.

Der gerade Schnitt dieses Trennvorganges ermöglicht auch das Herstellen dünner Probenscheiben (5b, 6b).

Die Trennschleifscheibe (32) ist an einer Nachstelleinrichtung (33) gelagert, durch die der Trennschleifscheibenverbrauch ausgeglichen werden kann. (Fig. 4)

In Fig. 6 bis 8 sind die verschiedenen Spektralproben gezeigt.

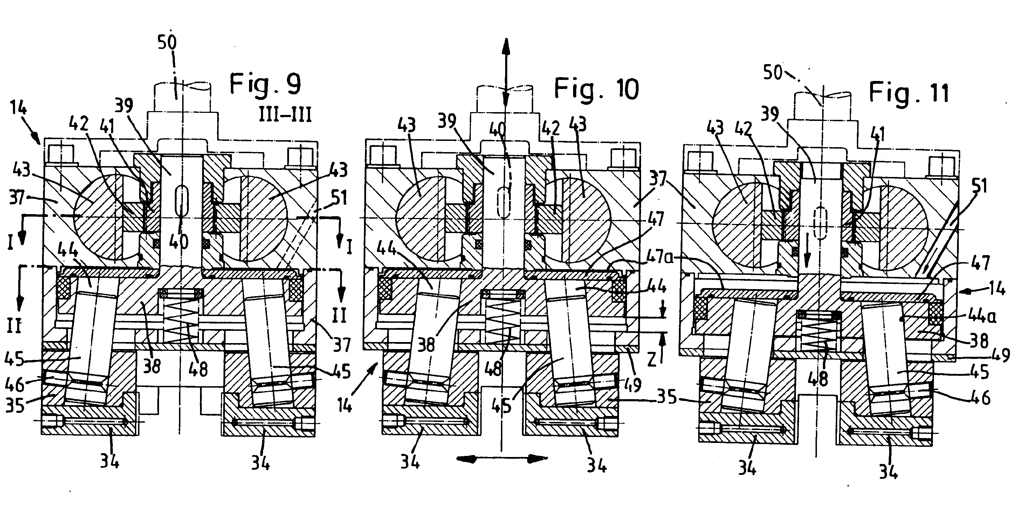

Die Spannvorrichtung (14), gemäß Fig. 9 bis 14, ist in Blockform ausgebildet und besitzt zwei in zwei Stufen gegeneinander bewegbare Spannbacken (34). Dabei sind die beiden Spannbacken (34) jeweils an einen Spannbackenträger (35) befestigt, mit dem sie in einer Führung (36) unter dem würfelförmigen, mehrteiligen Gehäuse (37) der Spannvorrichtung (14) aufeinanderzu und voneinander weg verschiebbar geführt sind.

In dem Gehäuse (37) ist ein Spannteller (38) axial drehbar gelagert, der mit seiner Drehachse (39) im oberen Gehäusebereich gelagert ist. Die Drehachse (39) steht über eine Nut-Feder-Verbindung (40) mit einem Zahnrad (41) in axialer Drehverbindung und ist gegenüber diesem Zahnrad (41) in einem begrenzten Bereich axial verschiebbar vorgesehen.

In das Zahnrad (41) greifen zwei Zahnstangen (42) ein, welche an Druckmittelzylindern (43) befestigt sind.

In dem Spannteller (38) sind zwei exzentrisch um die Teller-Drehachse (40) verlaufende Spannuten (44) ausgenommen, in die mit den Spannbacken (34) bewegungsstarr verbundene, die erste Spannstufe bei der Tellerverdrehung bewirkende Bolzen (45) eingreifen.

Dabei sind diese Bolzen (45) mit ihren unteren Längenenden in die Spannbackenträger (35) eingesteckt und darin durch Schrauben (46) od. dgl. fixiert. Die Spannuten (44) sind in dem Spannteller (38) schräg verlaufend ausgespart, d.h., sie erstrecken sich in Drehachsrichtung nach unten auseinanderdivergierend.

Die Bolzen (45) greifen kraftschlüssig in die Spannuten (44) ein.

Auf dem Spannteller (38) ist obenseitig ein Druckteller (47) angeordnet, der einerseits die Spannuten (44) überdeckt und andererseits eine druckbeaufschlagbare Kolbenfläche (47a) ergibt.

Eine Druckfeder (48) ist koaxial zur Drehachse (40) angeordnet und stützt sich einerseits im Spannteller (38) und andererseits auf einer zwischen Gehäuse (37) und Spannbackenträgern (35) angeordnete Widerlagerplatte (49) ab.

Das Gehäuse (37) der Spannvorrichtung (14) ist über vertikale Teleskopführungen (50) an dem Support (15) höhenverfahrbar gehalten.

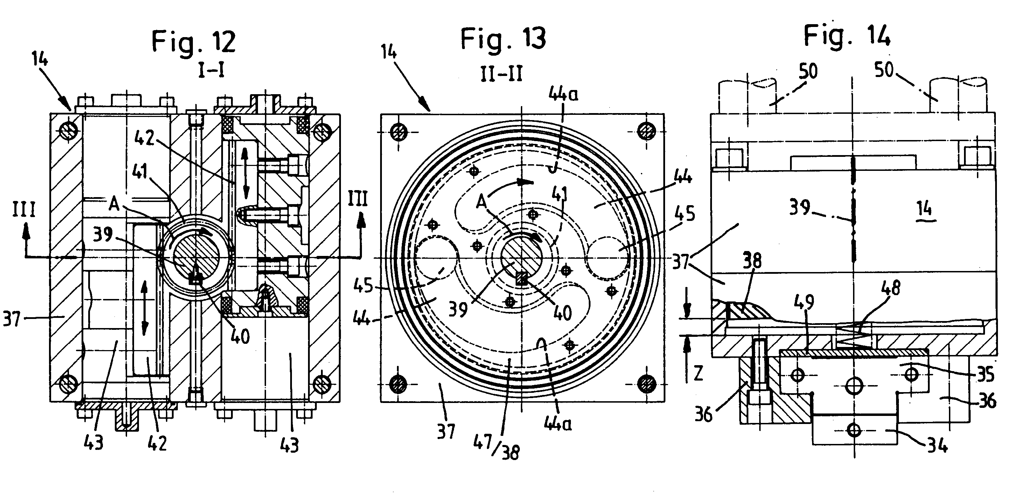

In Fig. 9 ist die geöffnete Stellung der Spannbacken (34) gezeigt und wie aus Fig. 12 ersichtlich sind die Zahnstangen (42) in entgegengesetzte Richtungen nach außen durch die Druckmittelzylinder (43) verfahren.

Fig. 13 zeigt ebenfalls die geöffnete Stellung der Spannvorrichtung (14), indem der Spannteller (38) in die Endstellung verdreht worden ist, in der die beiden Bolzen (45) in den am weitesten zur Drehachse (39) liegenden Nutenden sich befinden.

Durch Verschieben der beiden Zahnstangen (42) mittels der Druckmittelzylinder (43) aufeinanderzu wird das Zahnrad (41) und somit die damit gekuppelte Drehachse (40) in Pfeilrichtung "A" verdreht und dadurch erhält auch der Spannteller (38) seine Verdrehung in Pfeilrichtung "A". Hierdurch bewegen sich die beiden Nuten (44) um die Bolzen (45) und ziehen dabei die beiden Bolzen (45) aufeinanderzu und in Richtung Drehachse (39), da sich der Abstand der Nuten (44) zu der Drehachse (39) aufgrund des exzentrischen Nutverlaufes verringert.

Hierdurch werden die Spannbackenträger (35) und somit die Spannbacken (34) aufeinanderzu in die erste Spannstellung gemäß Fig. 10 zusammengefahren. In dieser verdrehten Spanntellerstellung liegen die Nuten (44) mit den anderen, der Drehachse (39) näher liegenden Längenenden mit in Drehrichtung gesehenem Abstand vor den Bolzen (45). Der Drehwinkel des Spanntellers (38) ist weniger als 90 Grad.

Danach erfolgt die Bewegung der Spannbacken (34) in die zweite Spannstufe, wobei durch einen Zufluß (51) (Druckmittelkanal) auf die Kolbenfläche (47a) des Drucktellers (47) ein Druckmedium eingebracht wird.

Die Kolbenfläche (47a) wird druckbeaufschlagt und durch diese Druckbeaufschlagung wird der Spannteller (38) in axialer Richtung nach unten verschoben, und zwar um das Maß (Z) in Fig. 10, wobei gleichzeitig die Drehachse (39) aufgrund der Nut-Federverbindung (40) sich gegenüber dem Zahnrad (41) ebenfalls axial verschieben kann.

Bei dieser Absenkbewegung des Spanntellers (38) wirkt die Keilfläche (44a) der Spannuten (44) auf die Bolzen (45) ein und bewirkt ein weiteres Zusammenschieben der Spannbacken (34) aufeinanderzu, und zwar so weit, bis die Bolzen (45) an die Nutenden anschlagen.

Dieser zweite Spannweg beträgt für jede Spannbacke (34) etwa 1 mm.

Bei etwa einem 10 mm Hub des Spanntellers (38) wird aufgrund der Keilfläche (44a) der Spannbackenhub von jeweils 1 mm erreicht.

Die Kolbenfläche (47a) ist verhältnismäßig groß und bei einer Druckbeaufschlagung von 4 bar wird eine Spannkraft von ca. 8.000 kp erreicht (1 bar = 10⁵ Pa und 1 kp = 9,81 N).

Durch die axiale Verdrehung des Spanntellers (38) und die nachfolgende axiale Verschiebung des Spanntellers (38) ist ein verhältnismäßig großer Spannweg der Spannbacken (34) erzielt worden, der bis zu 30 mm beträgt.

Die Druckfeder (48) bewirkt bei nachlassender Druckbeaufschlagung ein axiales Zurückschieben des Spanntellers (38) in die erste Spannstufe und danach erfolgt durch Verschieben der Zahnstangen (42) das Zurückdrehen des Spanntellers (38) und somit das Öffnen der Spannbacken (34).

## Patentansprüche

1. Aufbereitungssystem für Eisen- und Stahlproben, mit einer Schleifmaschine, einem Analysengerät und Transporteinrichtungen zur Ein-, Aus- und Übergabe der Proben, dadurch gekennzeichnet, daß
- die Schleifmaschine (1) mit Grob- und Feinschleifeinrichtung (18, 20), mindestens einer Kühleinrichtung (19) und einer Spannvorrichtung (14) für die Proben (4, 5, 6) als zentrales Modul mit
- einer ein Modul bildenden, einen integrierten Roboter (12) zur Übergabe der Proben in die jeweiligen einzelnen Bearbeitungsstationen, eine Sandstrahleinheit (26), eine induktive Erwärmstation (27), Probenbrecher (25) und mehrere Stanzen (28, 29, 30) für Laschenproben (4) beinhaltenden Stanzeinrichtung (2)
und mit
- einer ein Modul bildenden, eine aus rotierendem Spannfutter (31) und nachstellbarer Trennschleifscheibe (32) für zylindrische und konische Proben (5, 6) bestehende Trennstation aufweisenden Trennmaschine (3)
- zu einer modularen Baueinheit zusammengesetzt ist, und
- die Schleifmaschine (1) mit einem zentralen Entstaubungsanschluß (13) für die modulare Baueinheit ausgerüstet ist.

2. Aufbereitungssystem nach Anspruch 1 dadurch gekennzeichnet, daß die Schleifmaschine (1) mit einer Transporteinrichtung (7) für die Proben-Ein- und -Ausgabe, die Stanzeinrichtung (2) mit einer Transporteinrichtung (8) für die Proben-Ein- und -Ausgabe und die Trennmaschine (3) mit einer Transporteinrichtung (9) für die Probeneingabe versehen ist, zwischen Trennmaschine (3) und Stanzeinrichtung (2) und zwischen Stanzeinrichtung (2) und Schleifmaschine (1) je eine Transporteinrichtung (10, 11) für die Probenübergabe angeordnet sind, wobei alle Transporteinrichtungen (7 bis 11) von Transportbändern gebildet sind.

3. Aufbereitungssystem nach Anspruch 1, dadurch gekennzeichnet, daß die Spannvorrichtung (14) für die Proben (4, 5, 6) in der Schleifmaschine (1) in waagerechter und senkrechter Richtung bewegbar ist.

4. Aufbereitungssystem nach Anspruch 1, dadurch gekennzeichnet, daß die Grob- und die Feinschleifeinrichtung (18, 20) jeweils von endlos umlaufenden Schleifbändern (22, 23) gebildet ist, wobei die Proben (4, 5, 6) in der Grobschleifeinrichtung (18) oszillierend über das von einem Kontaktrad (21) angetriebene Schleifband (22) unter gleichzeitiger, schrittweiser Zustellung für die Schleiftiefe bewegbar und in der Feinschleifeinrichtung (20) unter Zustellung für die Schleiftiefe gegen das Schleifband (23) und einen darunter federnd gelagerten Schleiftisch (24) gehalten sind.

5. Aufbereitungssystem nach Anspruch 1, dadurch gekennzeichnet, daß die Grobschleifeinrichtung (18) eine an einer vertikalen Schleifspindel befestigte Topfschleifscheibe hat.

6. Aufbereitungssystem nach Anspruch 1, dadurch gekennzeichnet, daß die Spannvorrichtung (14) in Blockform ausgebildet ist und zwei in zwei Stufen gegeneinander bewegbare Spannbacken (34) besitzt.

7. Aufbereitungssystem nach Anspruch 6, dadurch gekennzeichnet, daß die Spannvorrichtung (14) einen über Druckmittelzylinder (43), Zahnstangen (42) und Zahnrad (41) axial verdrehbaren Spannteller (38) mit exzentrisch um die Spannteller-Drehachse (39) verlaufend ausgesparten Spannuten (44) hat, in die mit den Spannbacken (34) bewegungsstarr verbundene, die erste Spannstufe bei der Spanntellerverdrehung bewirkende Bolzen (45) eingreifen und der Spannteller (38) mit Bolzen (45) und Spannbacken (34) für die zweite Spannatufe durch Druckmittelbeaufschlagung axial verschiebbar ist.

8. Aufbereitungssystem nach Anspruch 6 und 7, dadurch gekennzeichnet, daß die beiden Spannbacken (34) jeweils an einen Spannbackenträger (35) befestigt sind, mit dem sie in einer Führung (36) unter dem würfelförmigen, mehrteiligen Gehäuse (37) der Spannvorrichtung (14) aufeinanderzu und voneinander weg verschiebbar geführt sind**.**

9. Aufbereitungssystem nach einem der Ansprüche 7 und 8, dadurch gekennzeichnet, daß der Spannteller (38) mit einer Drehachse (39) im oberen Gehäusebereich axial drehbar gelagert ist und mit seiner Drehachse (39) über eine Nut-Federverbindung (40) mit dem Zahnrad (41) in drehfester Verbindung und in axialer Verschiebeverbindung steht.

10. Aufbereitungssystem nach einem der Ansprüche 7 bis 9, dadurch gekennzeichnet, daß im oberen Bereich des Gehäuses (37) zwei Druckmittelzylinder (43) angeordnet sind, die jeweils eine mit dem Zahnrad (41) kämmende Zahnstange (42 aufweisen und durch Gegeneinanderverfahren der Zahnstange (42) die axiale Spanntellerverdrehung ergeben.

11. Aufbereitungssystem nach einen der Ansprüche 7 bis 10, dadurch gekennzeichnet, daß die beiden Spannuten (44) im Spannteller (38) schräg nach unten und außen divergierend ausgespart sind und mit ihrem, der Drehachse (39) am weitesten entfernten Nutenende die geöffnete Stellung der Spannbacken (34) und mit ihrem der Drehachse am nächsten liegenden Nutenende die Spannstellung der Spannbacken (34) begrenzen.

12. Aufbereitungsystem nach einem der Ansprüche 7 bis 11, dadurch gekennzeichnet, daß die beiden Bolzen (45) mit ihrem unteren Längenende in die Spannbackenträger (35) eingesteckt und durch Schrauben (46) od. dgl fixiert sind und mit ihrem oberen Längenende in die Spannuten (44) bewegbar und kraftschlüssig einfassen.

13. Aufbereitungssystem nach einem der Ansprüche 7 bis 12, dadurch gekennzeichnet, daß auf dem Spannteller (38) ein Druckteller (47) angeordnet ist, der die Spannuten (44) überdeckt und über einen Zufluß (51) durch ein Druckmittel für die axiale Spannteller-Verschiebung auf seiner, eine Kolbenfläche (47a) bildenden Oberseite beaufschlagbar ist.

14. Aufbereitungssystem nach einem der Ansprüche 7 bis 13, dadurch gekennzeichnet, daß koaxial zur Spannteller-Drehachse (39) eine Druckfeder (48) angeordnet ist, die sich einerseits im Spannteller (38) und andererseits auf einer zwischen Gehäuse (37) und Spannbackenträgern (35) angeordneten Widelagerplatte (49) abstützt. (49) abstützt.

15. Aufbereitungssystem nach einem der Ansprüche 7 bis 14, dadurch gekennzeichnet, daß die Spannuten (44) mit ihrer äußeren, schräg verlaufenden Nutfläche eine auf die Bolzen (45) einwirkende Keilfläche (44a) bilden.

16. Aufbereitungssystem nach einem der Ansprüche 7 bis 15, dadurch gekennzeichnet, daß die Spannvorrichtung (14) mittels Teleskopführungen an einem Support (15) in vertikaler Richtung höhenverfahrbar gehalten und mit dem Support (15) an waagerechten Führungen (16) oberhalb der Schleifeinrichtungen (18, 20) verfahrbar ist.

## Claims

1. System for preparing iron and steel specimens, comprising a grinding machine, an analyser, and conveying devices for feeding, unloading and transferring the specimens, characterised in that
- the grinding machine (1) is equipped with rough and fine grinding devices (18, 20), at least one cooling device (19) and a fixture (14) for the specimens (4, 5, 6) as a central module
with
- a punching device (2) constituting a module and containing an integrated robot (12) for transferring the specimens to the respective individual machining stations, a sand-blasting device (26), an inductive heating station (27), specimen breakers (25) and a plurality of punches (28, 29, 30) for bracket specimens (4)
and with
- a cutting-off machine (3) constituting a module and incorporating a cutting-off station consisting of a rotating chuck (31) and re-adjustable abrasive cutting-off wheel (32) for cylindrical and conical specimens (5, 6)
- is combined into a modular constructional unit,
and
- the grinding machine (1) is equipped with a central dust removal connection (13) for the modular constructional unit.

2. Preparation system according to claim 1, characterised in that the grinding machine (1) is provided with a conveyor device (7) for feeding and unloading the specimens, the punching device (2) with a conveyor device (8) for feeding and unloading the specimens, and the cutting-off machine (3) with a conveyor device (9) for feeding the specimens, a respective conveyor device (10, 11) is arranged between the cutting-off machine (3) and the punching device (2) and between the punching device (2) and the grinding machine (1) for transfer of the specimens, and all the conveyor devices (7 to 11) are constituted by conveyor belts.

3. Preparation system according to claim 1, characterised in that the fixture (14) for the specimens (4, 5, 6) is adapted to be horizontally and vertically movable in the grinding machine (1).

4. Preparation system according to claim 1, characterised in that the rough and fine grinding devices (18, 20) are each constituted by continuously circulating grinding belts (22, 23), the specimens (4, 5, 6) being held in the rough grinding device (18) in a manner adapted to be moved oscillatingly over the grinding belt (22), which is driven by a contact wheel (21), while simultaneously being adjusted stepwise for grinding depth, and being held in the fine grinding device (20) while being adjusted for grinding depth against the conveyor belt (23) and a grinding table (24) resiliently mounted therebelow.

5. Preparation system according to claim 1, characterised in that the rough grinding device (18) has a cup wheel secured to a vertical grinding spindle.

6. Preparation system according to claim 1, characterised in that the fixture (14) is in ingot form and possesses two clamping jaws (34) adapted to be moved towards one another in stages.

7. Preparation system according to claim 6, characterised in that the fixture (14) has a clamping plate (38), adapted to be turned axially by means of hydraulic cylinders (43), racks (42) and a gear (41) , with clamping grooves (44) recessed excentrically around the rotating shaft (39) of the clamping plate, into which grooves engage pins (45) immovably connected to the clamping jaws (34) and effecting the first clamping stage when the clamping plate is turned, and in that the clamping plate (38) is adapted to be axially displaced with the pins (45) and clamping jaws (34) by hydraulic action for the second clamping stage.

8. Preparation system according to claims 6 and 7, characterised in that the two clamping jaws (34) are respectively secured to a clamping jaw bracket (35) , with which they are adapted to be moved towards and away from one another in a guide (36) under the cubic, multi-part housing (37) of the fixture (14).

9. Preparation system according to either of claims 7 and 8, characterised in that the clamping plate (38) is mounted with a rotating shaft (39) in the upper portion of the housing so as to be axially rotatable and is connected by its rotating shaft (39) to the gear (41) via a tongue-and-groove joint (40) in non-rotatable but axially displaceable manner.

10. Preparation system according to any of claims 7 to 5, characterised in that in the upper portion of the housing (37) are disposed two hydraulic cylinders (43), each incorporating a rack (42) meshing with the gear (41) and producing axial clamping plate rotation by bringing together the rack (42).

11. Preparation system according to any of claims 7 to 10, characterised in that the two clamping grooves (44) recessed in the clamping plate (38) are angled downwards and diverge outwards, and with the end of the groove furthest from the rotating shaft (39) delimit the open position of the clamping jaws (34) and with the end of the groove nearest to the rotating shaft delimit the clamping position of the clamping jaws (34).

12. Preparation system according to any of claims 7 to 11, characterised in that the two pins (45) are pushed by their lower longitudinal ends into the clamping jaw brackets (35) and secured by screws (46) or similar and fit by their upper longitudinal ends movably and with force closure into the clamping grooves (44).

13. Preparation system according to any of claims 7 to 12, characterised in that disposed on the clamping plate (38) is a pressure plate (47) which covers the clamping grooves (44) and to which a pressure medium can be applied on its upper face, constituting a plunger face (47a), via an inflow (51) to produce axial displacement of the clamping plate.

14. Preparation system according to any of claims 7 to 13, characterised in that disposed coaxially to the clamping-plate rotating shaft (39) is a compression spring (48) which rests on the one hand in the clamping plate (38) and on the other hand on an abutment plate (49) disposed between the housing (37) and the clamping jaw brackets (35).

15. Preparation system according to any of claims 7 to 14, characterised in that the clamping grooves (44) constitute with their outer, angled groove surface a vee face (44a) which operates on the pins (45).

16. Preparation system according to any of claims 7 to 15, characterised in that the fixture (14) is held on a support (15) so as to be vertically height-adjustable by means of telescopic guides and is adapted to travel with the support (15) on horizontal guides (16) above the grinding devices (18, 20).

## Revendications

1. Système de préparation d'échantillons de fer et d'acier avec une meuleuse, un appareil d'analyse et des dispositifs de transport pour l'amenée, l'évacuation et le transfert des échantillons,
caractérisé par le fait que
- la meuleuse (1) est équipée de dispositifs de dégrossissage et de finition (18, 20), d'au moins un dispositif de refroidissement (19) et d'un dispositif de serrage (14) pour les échantillons (4, 5, 6) en tant que module central, assemblé
avec
- un dispositif d'estampage (2) formant un module et comprenant un robot intégré (12) pour le transfert des échantillons dans les différentes stations de traitement, une unité de sablage (26), une station de chauffage inductive (27), des concasseurs d'échantillons (25) et plusieurs estampeuses (28, 29, 30) pour échantillons d'éclisses (4)
et avec
- une machine à tronçonner (3) formant un module et comprenant une station de tronçonnage composée d'un mandrin de serrage rotatif (31) et d'une meule tronçonneuse (2) ajustable pour échantillons cylindriques et coniques (5, 6)
pour former une unité de construction modulaire,
et que
- la meuleuse (1) est pourvue d'un raccordement central de dépoussiérage (13) de l'unité de construction modulaire.

2. Système de préparation selon revendication 1, caractérisé par le fait
que la meuleuse (1) est pourvue d'un dispositif de transport (7) pour l'amenée et l'évacuation des échantillons, tandis que le dispositif d'estampage (2) est pourvu d'un dispositif de transport (8) pour l'amenée et l'évacuation des échantillons et la machine à tronçonner (3) d'un dispositif de transport (9) pour l'amenée des échantillons, un dispositif de transport (10, 11) étant prévu entre la machine à tronçonner (3) et le dispositif d'estampage (2), ainsi qu'entre le dispositif d'estampage (2) et la meuleuse (1) pour le transfert des échantillons, tous ces dispositifs de transport (7 à 11) consistant en bandes transporteuses.

3. Système de préparation selon revendication 1, e de préparation selon revendication 1, caractérisé par le fait
que le dispositif de serrage (14) pour les échantillons (4, 5, 6) est mobile horizontalement et verticalement dans la meuleuse (1).

4. Système de préparation selon revendication 1, caractérisé par le fait
que les dispositifs de dégrossissage et de finition (18, 20) sont formés, respectivement, par des bandes abrasives (22, 23) tournant en continu, les échantillons (4, 5, 6) se mouvant en oscillant, dans le dispositf de dégrossissage (18), sur la bande abrasive (22) entraînée par une roue de contact (21), l'avance concernant la profondeur de meulage ayant lieu simultanément, pas à pas, tandis que, dans le dispositif de finition (20), les échantillons (4, 5, 6) sont maintenus contre la bande abrasive (23) et une table de meulage (24), située dessous, et montée élastiquement.

5. Système de préparation selon revendication 1, caractérisé par le fait
que le dispositifs dégrossissage (18) est pourvu d'une meule-boisseau fixée à une broche porte-meule verticale.

6. Système de préparation selon revendication 1, caractérisé par le fait
que le dispositif de serrage (14) est conçu en forme de bloc et pourvu de deux mâchoires de serrage (34) se déplaçant l'une vers l'autre en deux phases.

7. Système de préparation selon revendication 6, caractérisé par le fait
que le dispositif de serrage (14) possède un plateau de serrage (38) pivotable axialement par l'intermédiaire d'un cylindre compresseur (43), de crémaillères (42) et d'une roue dentée (41), le plateau de serrage (38) présentant des gorges (44), qui s'étendent excentriquement autour du pivot (39) du plateau de serrage (38) et dans lesquelles s'enclenchent les goupilles (45) reliées rigidement aux mâchoires de serrage (34), ce dont résulte la premiére phase de serrage, et le plateau de serrage (38) étant déplaçable axialement, avec les goupilles (45) et les mâchoires, sous l'action du fluide comprimé, lors de la seconde phase de serrage.

8. Système de préparation selon revendications 6 et 7,
caractérisé par le fait
que les deux mâchoires de serrage (34) sont fixées chacune à un porte-mâchoires (35), avec lequel elles sont conduites l'une vers l'autre et l'une éloignées l'une de l'autre, dans un guide (36) situé sous le carter cubique (37), composé de plusieurs piéces, du dispositif de serrage (14).

9. Système de préparation selon revendications 7 et 8,
caractérisé par le fait
que le plateau de serrage (38) est monté dans la partie supérieure du carter avec un pivot (39), autour duquel et par lequel il est relié avec la roue dentée (41) par assemblage à rainure et languette, résistant à la torsion et permettant un déplacement axial.

10. Système de préparation selon l'une des revendications 7 à 9, caractérisé par le fait
que, dans la partie supérieure du carter (37), deux cylindres compresseurs (43) sont disposés, lesquels présentent, chacun, une crémaillère (42) coopérant avec la roue dentée (41), ce dont résulte le pivotement axial du plateau de serrage par.

11. Système de préparation selon l'une des revendications 7 à 10,
caractérisé par le fait
que les deux rainures de serrage (44) sont pratiquées obliquement, en divergeant vers le bas et l'extérieur, dans le plateau de serrage (38), leur extrémité la plus éloignée du pivot (39) limitant la position ouverte des mâchoires de serrage (34) et leur extrémité la plus proche du pivot limitant la position de serrage des mâchoires de serrage (34).

12. Système de préparation selon l'une des revencations 7 à 11,
caractérisé par le fait
que les deux goupilles (45) sont enfichées par leur extrémité longitudinale, inférieure dans les supports (35) des mâchoires de serrage et fixées à l'aide de vis (46) ou autre moyen analogue, tandis que leur extrémité longitudinale, supérieure s'enclenche, mobile, par adhérence, dans les rainures (44).

13. Systéme de préparation selon l'une des revendications 7 à 12,
caractérisé par le fait
qu'un plateau de compression (47), qui, installé sur le plateau de serrage (38), recouvre les rainures (44) et dont la face supérieure, en forme de piston (47a), est soumise à l'action d'un fluide, passant par l'amenée (51) et assurant le déplacment axiale du plateau de serrage (38).

14. Système de préparation selon l'une des revendications 7 à 13,
caractérisé par le fait
qu'un ressort de pression (48) est disposé coaxialement par rapport au pivot (39) du plateau de serrage (38) et prend appui, d'une part, dans le plateau de serrage (38) et, d'autre part, sur une plaque de butée (49), située entre le carter (37) et les supports (35) des mâchoires.

15. Système de préparation selon l'une des revendications 7 à 14,
caractérisé par le fait
que la surface extérieure, oblique des rainures de serrage (44) forme une cale (44a) agissant sur les goupilles (45).

16. Système de préparation selon l'une des revendications 7 à 15,
caractérisé par le fait
que le dispositif de serrage (14) est maintenu, mobile verticalement, sur un support (15) à l'aide de guides télescopiques et peut être déplacé, avec le support (15), sur des guides horizontaux (16), au-dessus des dispositifs de meulage (18, 20).
